# EUROPÄISCHE PATENTSCHRIFT

(11) **EP 1 512 965 B1**
(45) Veröffentlichungstag und Bekanntmachung des Hinweises auf die Patenterteilung: **25.11.2009**
(21) Anmeldenummer: 04019140.5
(22) Anmeldetag: 12.08.2004
(51) Int. Cl.: G01N 25/56

(54) **Verfahren und Anordnung zur Messung der Wasseraktivität**
Method and arrangement for measuring water acitivity
Procédé et agencement pour mesurer l'activité de l'eau

(30) Priorität: 14.08.2003 DE 10337306
(43) Veröffentlichungstag der Anmeldung: 09.03.2005
(73) Patentinhaber: IL Metronic Sensortechnik GmbH, 98693 Ilmenau-Unterpörlitz (DE)
(72) Erfinder: Heinze, Dirk, Prof. Dr.-Ing., 98693 Ilmenau (DE); Bürger, Heinz-Dieter, 97877 Wertheim (DE); Altstadt, Erhard, Dr.-Ing., 98693 Oberpölitz (DE)
(74) Vertreter: Liedtke, Klaus

(56) Entgegenhaltungen:
- US-A- 5 189 902
- ZANONI B ET AL: "Design and setting up of a water vapour pressure capacitance manometer for measurement of water activity" J FOOD ENG; JOURNAL OF FOOD ENGINEERING DEC 1998 ELSEVIER SCI LTD, EXETER, ENGL, Bd. 38, Nr. 4, Dezember 1998 (1998-12), Seiten 407-423, XP002306832
- KHAN SHAHNAZ A ET AL: "Measurement and control of water activity with an aluminium oxide sensor in organic two-phase reaction mixtures for enzymic catalysis" ENZYME MICROB TECHNOL; ENZYME AND MICROBIAL TECHNOLOGY JUN 1990, Bd. 12, Nr. 6, Juni 1990 (1990-06), Seiten 453-458, XP002306833

## Beschreibung

Die Erfindung betrifft ein Verfahren und eine Anordnung zur Messung der Wasseraktivität a_{w}.

Die Erfindung wird vorzugsweise angewandt, um die Wasseraktivität von Lebensmitteln zu bestimmen und so Informationen über deren Haltbarkeit und/oder Reifungsvorgang zu gewinnen.

Eine Einführung in die Thematik der Wasseraktivität bietet das Fachbuch "Industrielle Feuchtemessung" von Roland Wemecke, erschienen 2003 im Wiley-VCH-Verlag auf den Seiten 249-268 oder das Kapitel 64 "Wasseraktivität" des Schweizerischen Lebensmittelbuches, einsehbar beispielsweise unter http://www.bag.admin.ch. Danach wird unter der Wasseraktivität einer Probe der Quotient aus dem Wasserdampfdruck über der Oberfläche der Probe und dem Wasserdampfdruck über reinem Wasser bei gleicher Temperatur wie die der Probe, also dem Sättigungsdampfdruck, verstanden. Im Gleichgewicht entspricht das der relativen Feuchte über der Probenoberfläche.

Im Stand der Technik sind bereits Verfahren und Anordnungen bekannt, um die Wasseraktivität einer Probe zu bestimmen. Sie beruhen meist darauf, aus der mit Feuchtesensoren bestimmten Gleichgewichtsfeuchte und der gemessenen Temperatur in einer abgeschlossenen Probenkammer die Wasseraktivität zu berechnen.

In US 40 15 463 sowie in DE 24 31 051 C2 wird eine portable Vorrichtung zur Messung der Wasseraktivität mit einem Hygrometer und einem Thermometer beschrieben. Nachteilig daran sind lange Messzeiten und die damit verbundene, beschränkte Akzeptanz für Qualitätskontrollen.

Ferner ist nach US 42 15 568 ein Gerät zur schnellen Bestimmung der Wasseraktivität bekannt, in dem ein geschlossener Pumpkreislauf mit einem optischen Taupunkthygrometer zur Bestimmung der Gleichgewichtsfeuchte eingesetzt wird. Dabei ist nachteilig, dass die optische Fläche des Taupunkthygrometers durch Rückstände häufig verschmutzt wird.

In JP 03 14 4354 A ist ein Feuchtesensor zur Messung der Wasseraktivität angegeben, der die Form einer Nadel hat und in die Probe hineingesteckt wird. Diese Vorrichtung muss nach jeder Anwendung gründlich gereinigt werden, um Messfehler zu verhindern. Außerdem ist eine regelmäßige Eichung erforderlich.

In EP 0 297 415 A2 werden ein Verfahren und eine Vorrichtung zur Bestimmung der Wasseraktivität in Lebensmitteln, insbesondere Fleischerzeugnissen, beschrieben, bei denen als Repräsentation der Wasseraktivität der Gefrier- oder Schmelzpunkt der Probe benutzt wird. Nachteilig daran ist, dass der Gefrier- bzw. Schmelzvorgang relativ lange dauert und die Messung des Gefrier- bzw. Schmelzpunktes ungenau ist.

Die US 58 16 704 beschreibt ein Gerät und ein Verfahren zur Messung der Wasseraktivität und Taupunkttemperatur, in der eine schnellere Einstellung des Gleichgewichts durch Verwirbelung der Probenatmosphäre und Lenkung eines Luftstromes auf ein Taupunkhygrometer bewirkt werden soll. Von Nachteil ist dabei, dass die Kondensationsfläche des Taupunkthygrometers auch hier ständiger Verschmutzung ausgesetzt ist.

In dem Aufsatz "Design and Setting up of a Water Vapour Pressure Capacitance Manometer for Measurement of Water Activity" in Journal of Food Engineering 38 (1999) auf den Seiten 407-423 werden Messungen des Wasserdampfdruckes unter Vakuum und der Temperatur beschrieben, aus denen die Wasseraktivität bestimmt wird. Nachteilig dabei ist, dass abhängig von den Pumpzeiten mehr oder weniger der Partialdruck von Permanentgasen, die aus der Probe und von den Wandungen und Spalten der Messvorrichtung abgegeben werden, den Wasserdampfdruck überlagert und damit die Berechnung der Wasseraktivität störend beeinflussen bzw. zeitaufwendig machen.

Es sind ferner Vakuummessgeräte nach dem Wärmeleitungsprinzip nach Pirani bekannt, die einen weiten Arbeitsbereich aufweisen, so z.B. ein MicroPirani von der Fa. Wenzel Instruments, Dänemark, mit einen Messbereich von 10⁻⁵ mbar bis 10⁺³ mbar.

Der Erfindung liegt die Aufgabe zugrunde, eine Anordnung und ein schnell und genau durchführbares Verfahren für die Messung der Wasseraktivität anzugeben, wobei der störende Einfluss von Permanentgasen vermieden wird und die oben genannten Nachteile gemindert werden oder nicht auftreten.

Erfindungsgemäß wird die Aufgabe durch ein Verfahren, welches die in Anspruch 1 angegebenen Merkmale aufweist, und mit einer Anordnung, welche die in Anspruch 5 angegebenen Merkmale aufweist, gelöst.

Vorteilhafte Ausgestaltungen sind Gegenstand der Unteransprüche.

Dabei kann vorteilhaft in der Abpumpeinrichtung ein Regel-/Absperr-Ventil und/oder eine Vakuumpumpe so geregelt werden, dass der Druck in der evakuierten Probenkammer nahezu ausschließlich durch den Partialdruck des Wasserdampfes bestimmt wird.

Bei einer gesonderten Ausführung wird die Evakuierung der Atmosphäre unterbrochen, wenn ein zusätzlicher Temperatursensor, der die Temperatur nahe der Probenoberfläche misst, eine andere Temperatur ermittelt als der Temperatursensor, der die Atmosphärentemperatur der Probenkammer misst. Bevorzugt erfolgt die Unterbrechung dann, wenn die Temperatur an der Probenoberfläche um 0,2 K bis 0,4 K unter der Atmosphärentemperatur der Probenkammer liegt.
Üblicherweise wird die Messung abgeschlossen, wenn keine Temperaturdifferenz zwischen den beiden Temperatursensoren vorliegt.

Die erfindungsgemäße Anordnung verfügt über einen Temperatursensor und eine regelbare Abpumpeinrichtung, einen Gesamtdrucksensor, der den Gesamtdruck der Kammeratmosphäre misst, sowie einen weiteren Drucksensor, der durch eine von dem ersten Drucksensor verschiedene Wirkungsweise auch die Existenz anderer Gase als Wasserdampf in der Probenkammer anzeigt, und eine Ansteuereinheit, die mit dem Gesamtdrucksensor, dem weiteren Sensor und dem Temperatursensor verbunden ist.
Bei vorteilhaften Ausführungen enthält die Abpumpeinrichtung ein Regel-/Absperr-Ventil und/oder eine regelbare Vakuumpumpe.
Ferner ist es möglich, dass durch die Probenkammer ein zusätzlicher
Temperatursensor durchgeführt ist, der bevorzugt 0,5 mm bis 2 mm von der Probenoberfläche entfernt angeordnet ist.
Zur Darstellung der Messergebnisse enthält die Anordnung eine Anzeigeeinheit.

Die Ausgestaltungen der Erfindung weisen eine Reihe von Vorteilen auf. Hierzu gehören insbesondere:
1. Durch die Verwendung einer regelbaren Abpumpeinheit für die Messung des Wasserdampfdruckes und damit für die Bestimmung der Wasseraktivität werden günstige zeitliche Druckverläufe in der Probenkammer erzielt.
2. Die Verwendung zweier Drucksensoren unterschiedlicher Wirkungsweise, wovon der eine speziell auf den Absolutdruck von Wasserdampf geeicht ist, birgt den Vorteil, dass unterschiedliche Messwerte der beiden Drucksensoren auf die Anwesenheit von anderen Gasen und/oder Dämpfen neben Wasserdampf hinweisen.
3. Durch einen zweiten Temperatursensor in der Nähe der Probe kann bei abfallender Temperatur ein übermäßiges, messwertverfälschendes Verdampfen von Wasser aus der Probe infolge des Auftretens von Verdunstungskälte erkannt und damit unterdrückt werden kann, was insbesondere bei Proben in Pulverform mit hoher Wasseraktivität bedeutsam ist.
4. Durch das erfindungsgemäße Verfahren und die erfindungsgemäße Vorrichtung werden die Messzeiten automatisch optimiert, die Messgenauigkeit erhöht und die Akzeptanz der Messungen auch auf problematische Substanzen erweitert.

Die Erfindung wird im Folgenden anhand eines Ausführungsbeispiels näher erläutert.

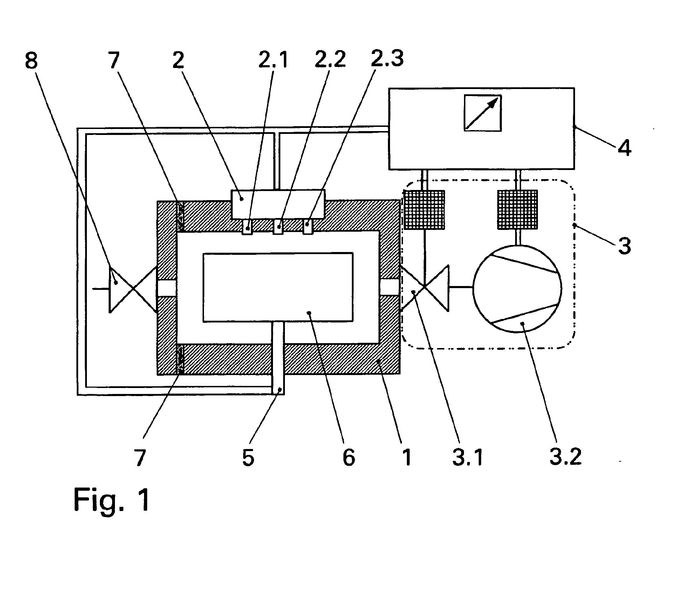

Dazu zeigt **Figur 1** eine schematische Darstellung einer erfindungsgemäßen Messanordnung.

In einer ersten Ausführung ist eine verschließbare Probenkammer 1 aus vernickeltem Aluminium mit einem Innenvolumen von 20 cm³ bis 50 cm³ mit Flachdichtungen 7 aus HTV-Silikon und einem Absperrventil 8 ausgestattet. Der Innenraum ist damit UHV-gemäß gestaltet. Die Vorrichtung enthält ferner eine Sensoranordnung 2, bestehend aus einem Gesamtdrucksensor 2.1, einen Drucksensor 2.2 gemäß dem Prinzip der Messung der Wärmeleitung nach Pirani sowie einen Temperatursensor 2.3. Der Innenraum ist mit einer Abpumpeinrichtung 3 verbunden, welche ein spaltfrei angeschlossenes Absperr-/Regel-Ventil 3.1 und eine Vakuumpumpe 3.2 mit einem Enddruck von 5 · 10⁻² mbar oder besser enthält. Der Durchlass des Absperr-/Regel-Ventils 3.1 und die Drehzahl der Vakuumpumpe 3.2 werden durch die Ansteuer- und Ausgabeeinheit 4 geregelt, die ihrerseits mit den Ausgängen der Sensoranordnung 2 verbunden ist. Das Absperrventil 8 ist zur Belüftung der Probenkammer 1 nach Beendigung der Messung vorgesehen.

Für die Ermittlung des a_{w}-Wertes wird folgendermaßen verfahren: Die Probenkammer 1 wird mit einem Anfangssaugvermögen von 0,2 m³/h bis 2 m³/h evakuiert. Sobald sich der anfängliche zeitliche logarithmische Druckabfall um mehr als den Faktor 1,1 bis 1,3 verkleinert, wird der weitere Druckverlauf so geregelt, dass die relative Druckänderung in einem Korridor von - 0,05 / min und + 0,01 / min verbleibt. Dies erfolgt vorzugsweise durch Drosselung des Absperr-/Regel-Ventils 3.1 und der Vakuumpumpe 3.2. Wenn unter diesen Regelbedingungen der Messwert des Drucksensors 2.2, der auf eine Absolutdruckmessung von reinem Wasserdampf abgeglichen ist, nur noch eine relative Druckänderung in den Schranken von ± 0,005 / min und eine Abweichung von weniger als 0,5% zum Messwert des Gesamtdruckmessers 2.1 aufweist, wird aus dem Messwert des Drucksensors 2.2 und der Temperatur der Probenkammer 1, die mit dem Temperatursensor 2.3 der Sensoranordnung 2 gemessen wird, der a_{w}-Wert durch die Ansteuer- und Ausgabeeinheit 4 errechnet und zur Anzeige gebracht.

In einer weiteren Ausführung ist durch die Probenkammer 1 ein zusätzlicher Temperatursensor 5 durchgeführt, der mit einem Abstand von 0,5 mm bis 2 mm an die Oberfläche der Probe 6, die in der Probenkammer 1 eingeschlossen ist, geführt ist. In das vorstehend beschriebene Messverfahren greift der zusätzliche Temperatursensor 5 dann ein, wenn sein Messwert eine temporäre Abweichung von mehr als - 0,2 bis - 0,4 K vom Messwert des Temperatursensors 2.3 aufweist. Der Eingriff erfolgt dergestalt, dass das Saugvermögen der Abpumpeinrichtung 3 auf Null gedrosselt wird. Das Verschwinden dieser temporären Abweichung ist eine weitere Bedingung für die vorstehend beschriebene Anzeige des a_{w}-Wertes.

### BEZUGSZEICHENLISTE

- 1: Probenkammer
- 2: Sensoranordnung
- 2.1: Gesamtdrucksensor
- 2.2: Drucksensor
- 2.3: Temperatursensor
- 3: Abpumpeinrichtung
- 3.1: Absperr-/Regelventil
- 3.2: Vakuumpumpe
- 4: Ansteuer- und Ausgabeeinheit
- 5: zusätzlicher Temperatursensor
- 6: Probe
- 7: Dichtungen
- 8: Absperrventil

## Patentansprüche

1. Verfahren zur Messung der Wasseraktivität einer Probe (6) in einer isolierten Probenkammer (1) mit einem Temperatursensor (2.3) und einer Abpumpeinrichtung (3), wobei eine Probenkammer (1) mit einem Anfangssaugvermögen von 0,2 m³/h bis 2 m³/h evakuiert wird und sobald sich der anfängliche zeitliche logarithmische Druckabfall um mehr als den Faktor 1,1 bis 1,3 verkleinert der weitere Druckverlauf so geregelt wird, dass die relative Druckänderung in einem Korridor von - 0,05 / min und + 0,01 / min verbleibt, wobei dann, wenn unter diesen Regelbedingungen der Messwert eines Drucksensors (2.2), der auf eine Absolutdruckmessung von reinem Wasserdampf abgeglichen ist, nur noch eine relative Druckänderung in den Schranken von + 0,005 / min und eine Abweichung von wenige als 0,5% zum Messwert eines Gesamtdruckmessers (2.1) aufweist, und wobei der Gesamtdruckmesser (2.1) und der Drucksensor (2.2) verschiedene Wirkungsweisen aufweisen, aus dem Messwert des Drucksensors (2.2) und der Temperatur der Probenkammer (1), die mit den Temperatursensor (2.3) gemessen wird, der wasserktivitats-Wert durch eine Ansteuer- und Ausgabeeinheit (4) errechnet und zur Anzeige gebracht wird.

2. Verfahren nach Anspruch 1, **dadurch gekennzeichnet, dass** die Evakuierung der Atmosphäre unterbrochen wird, wenn ein zusätzlicher Temperatursensor (5), der die Temperatur nahe der Probenoberfläche misst, eine andere Temperatur ermittelt als der Temperatursensor (2.3), der die Atmosphärentemperatur der Probenkammer (1) misst.

3. Verfahren nach Anspruch 2, **dadurch gekennzeichnet, dass** die Unterbrechung dann erfolgt, wenn die Temperatur an der Probenoberfläche um 0,2 K bis 0,4 K unter der Atmosphärentemperatur der Probenkammer (1) liegt.

4. Verfahren nach Anspruch 2 oder 3, **dadurch gekennzeichnet, dass** die Messung abgeschlossen wird, wenn keine Temperaturdifferenz zwischen den beiden Temperatursensoren (2.3, 5) vorliegt.

5. Anordnung zur Messung der Wasseraktivität einer Probe (6) in einer thermisch isolierten Probenkammer (1) mit einem Temperatursensor (2.3) und einer Abpumpeinrichtung (3), insbesondere zur Durchführung des Verfahrens nach einem der vorhergehenden Ansprüche, wobei die Anordnung
- eine Sensoranordnung (2), bestehend aus einem Gesamtdrucksensor (2.1), der den Gesamtdruck der Kammeratmosphäre misst, einem weiteren Drucksensor (2.2), gemäß dem Prinzip der Wärmeleitung nach Pirani, der auf eine Absolutdruckmessung von reinem wasserdampf abgeglichen ist, sowie einem Temperatursensor (2.3), wobei Gesamtdruckmesser (2.1) und Drucksensor (2.2) verschiedene Wirkungsweisen aufweisen und
- eine Ansteuer- und Ausgabeeinheit (4) zur Drehzahlregelung einer Vakuumpumpe (3.2), wobei die Ansteuer- und Ausgabeeinheit (4) geeignet ist, aus dem Messwert des Drucksensors (2.2) und dem Messwert des Temperatursensors (2.3) der Wert der Wasseraktivität (a_{w}-Wert) zu errechnen und zur Anzeige zu bringen und wobei die Ansteuer- und Ausgabeeinheit (4) mit den Ausgängen der Sensoranordnung (2) verbunden ist,
enthält.

6. Anordnung nach Anspruch 5, **dadurch gekennzeichnet, dass** die Abpumpeinrichtung (3) ein Regel-/Absperr-Ventil (3.1) und/oder einen zusätzlichen Temperatursensor (5) enthält.

7. Anordnung nach Anspruch 5 oder 6, **dadurch gekennzeichnet, dass** der zusätzliche Temperatursensor (5) 0,5 mm bis 0,2 mm von der Probenoberfläche entfernt angeordnet ist.

8. Anordnung nach einem der Ansprüche 5 bis 7, **dadurch gekennzeichnet, dass** die Anordnung eine Anzeigeeinheit (4) enthält.

## Claims

1. Method for measuring the water activity of a sample (6) in an insulated sample chamber (1) with a temperature sensor (2.3) and a mechanism (3) for pumping down, wherein a sample chamber (1) is evacuated with an initial suction capability of 0.2 m³/h to 2 m³/h, wherein, as soon as the initial temporal logarithmic pressure drop decreases by a factor of 1.1 to 1.3, the consecutive pressure course is controlled in a manner to keep the relative pressure variation in a range from - 0.05 / min to + 0.01 / min, wherein, provided the measured value of a pressure sensor (2.2), calibrated to an absolute value pressure measurement of pure water vapour, merely exhibits a relative pressure variation within the limits of + 0,005 / min and a variation of less than 0.5% with respect to the measured value of a total pressure gauge (2.1) under these controlling conditions and wherein the total pressure gauge (2.1) and the pressure sensor (2.2) exhibit distinct modes of action, the water activity value is calculated from the measured value of the pressure sensor (2.2) and the temperature of the sample chamber (1), which is measured by the temperature sensor (2.3), and displayed by means of a control- and display unit (4).

2. Method according to claim 1, **characterized in that** the evacuation of the atmosphere is interrupted if an additional temperature sensor (5) measuring the temperature near the sample surface, determines another temperature than the temperature sensor (2.3) measuring the atmosphere's temperature in the sample chamber (1).

3. Method according to claim 2, **characterized in that** the interruption is carried out if the temperature at the sample surface is 0.2 K to 0.4 K below the atmosphere's temperature in the sample chamber (1).

4. Method according to claim 2 or 3, **characterized in that** the measurement is terminated if no temperature difference is existent between both temperature sensors (2.3, 5).

5. Arrangement for measuring the water activity of a sample (6) in a thermally insulated sample chamber (1) with a temperature sensor (2.3) and a mechanism (3) for pumping down, in particular for carrying out the method according to one of the preceding claims, wherein the arrangement contains:
- a sensor arrangement (2), consisting of a total pressure sensor (2.1) measuring the total pressure of the chamber's atmosphere, another pressure sensor (2.2) according to the principle of heat conduction according to Pirani, which is calibrated to an absolute value pressure measurement of pure water vapour, and a temperature sensor (2.3), wherein the total pressure gauge (2.1) and the pressure sensor (2.2) exhibit different modes of action, and
- a control- and display unit (4) for controlling the rotation speed of a vacuum pump (3.2), wherein the control- and display unit is able to calculate and display the value of the water activity (a_{w} value) from the measured value of the pressure sensor (2.2) and the measured value of the temperature sensor (2.3), and wherein the control and display unit (4) is connected to the outputs of the sensor arrangement (2).

6. Arrangement according to claim 5, **characterized in that** the mechanism (3) for pumping down contains a control- / cut-off valve (3.1) and/or an additional temperature sensor (5).

7. Arrangement according to claim 5 or 6, **characterized in that** the additional temperature sensor (5) is arranged at a distance of 0.5 mm to 0.2 mm from the sample surface.

8. Arrangement according to one of the claims 5 to 7, **characterized in that** the arrangement comprises a display unit (4).

## Revendications

1. Procédé de mesure de l'activité aqueuse d'un échantillon (6) dans une chambre d'échantillonnage (1) isolée avec un capteur de température (2.3) et un dispositif de pompage (3), où le vide est fait dans une chambre d'échantillonnage (1) avec une capacité d'aspiration initiale de 0,2 m³/h à 2 m³/h, où, dès que la chute de pression temporelle logarithmique initiale est diminuée d'un facteur supérieur à 1,1 à 1,3, la courbe de pression suivante est régulée de telle manière que la variation relative de pression reste dans un couloir entre - 0,05 / min et + 0,01 / min, où, dans ces conditions de régulation, la valeur de mesure d'un capteur de pression (2.2) lissée à la mesure de pression absolue de vapeur d'eau pure, ne présente alors qu'une variation relative de pression entre les limites de + 0,005 / min et un écart inférieur à 0,5% par rapport à la valeur de mesure d'un indicateur de pression totale (2.1), où l'indicateur de pression totale (2.1) et le capteur de pression (2.2) présentent des modes de fonctionnement différents, et où, à partir de la valeur de mesure du capteur de pression (2.2) et de la température de la chambre d'échantillonnage (1) mesurée par le capteur de température (2.3), la valeur d'activité aqueuse est calculée par une unité de commande et de sortie (4) et affichée.

2. Procédé selon la revendication 1, **caractérisé en ce que** le pompage de l'atmosphère est interrompu si un capteur de température additionnel (5), qui mesure la température à proximité de la surface d'échantillon, saisit une température différente de celle du capteur de température (2.3) mesurant la température d'atmosphère de la chambre d'échantillonnage (1).

3. Procédé selon la revendication 2, **caractérisé en ce que** l'interruption est effectuée si la température à la surface d'échantillon est inférieure de 0,2 K à 0,4 K à la température d'atmosphère de la chambre d'échantillonnage (1).

4. Procédé selon la revendication 2 ou la revendication 3, **caractérisé en ce que** la mesure est terminée si aucune différence de température n'est présentée entre les deux capteurs de température (2.3, 5).

5. Dispositif de mesure de l'activité aqueuse d'un échantillon (6) dans une chambre d'échantillonnage (1) isolée avec un capteur de température (2.3) et un dispositif de pompage (3), en particulier pour l'exécution du procédé selon l'une des revendications précédentes, où le dispositif comprend
- un dispositif de capteur (2), composé d'un capteur de pression totale (2.1) qui mesure la pression totale de l'atmosphère de chambre, d'un autre capteur de pression (2.2) suivant le principe de conduction de chaleur de Pirani, lissé à une mesure de pression absolue de vapeur d'eau pure, ainsi que d'un capteur de température (2.3), l'indicateur de pression totale (2.1) et le capteur de pression (2.2) présentant des modes de fonctionnement différents
et
- une unité de commande et de sortie (4) pour la régulation de régime d'une pompe à vide (3.2), ladite unité de commande et de sortie (4) étant apte à calculer la valeur d'activité aqueuse (valeur a_{w}) à partir de la valeur de mesure du capteur de pression (2.2) et de la valeur de mesure du capteur de température (2.3), et à afficher celle-ci, et ladite unité de commande et de sortie (4) étant reliée aux sorties du dispositif de capteur (2).

6. Dispositif selon la revendication 5, **caractérisé en ce que** le dispositif de pompage (3) comprend une vanne de régulation/de fermeture (3.1) et/ou un capteur de température additionnel (5).

7. Dispositif selon la revendication 5 ou la revendication 6, **caractérisé en ce que** le capteur de température additionnel (5) est distant de 0,5 mm à 0,2 mm de la surface d'échantillon.

8. Dispositif selon l'une des revendications 5 à 7, **caractérisé en ce que** le dispositif comprend une unité d'affichage (4).
